(19) Europäisches Patentamt
European Patent Office
Office européen des brevets

(11) Numéro de publication: **0 129 474**
**B1**

(12) **FASCICULE DE BREVET EUROPEEN**

(45) Date de publication du fascicule du brevet: **23.04.86**

(51) Int. Cl.⁴: **C 07 C 6/04**, B 01 J 31/02 // C07C67/333

(21) Numéro de dépôt: **84401213.8**

(22) Date de dépôt: **14.06.84**

(54) **Catalyseur perfectionné pour la métathèse d'oléfines.**

(30) Priorité: **15.06.83 FR 8309876**
**08.06.84 FR 8409001**

(43) Date de publication de la demande:
**27.12.84 Bulletin 84/52**

(45) Mention de la délivrance du brevet:
**23.04.86 Bulletin 86/17**

(84) Etats contractants désignés:
**CH DE GB IT LI NL**

(56) Documents cités:
**FR - A - 2 180 038**

**INFORMATIONS CHIMIE, no. 201, mai 1980, J. OTTON:"La réaction de métathèse (ou dismutation) des oléfines", pages 161-168**

(73) Titulaire: **SOCIETE NATIONALE ELF AQUITAINE, Tour Aquitaine, F-92400 Courbevoie (FR)**

(72) Inventeur: **Basset, Jean, Marie, Maurice, 21 Petite rue de la Doua, F-69100 Villeurbanne (FR)**
Inventeur: **Leconte, Michel, 26 rue Jean-Claude Vivant, F-69100 Villeurbanne (FR)**
Inventeur: **Ollivier, Jean, Croix de Buzy, F-64260 Arudy (FR)**
Inventeur: **Quignard, Françoise, 15 rue du Chariot d'Or, F-69004 Lyon (FR)**

(74) Mandataire: **Kohn, Armand, 5 Avenue Foch, F-92380 Garches (FR)**

EP 0 129 474 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne un catalyseur perfectionné pour la métathèse de différentes oléfines; elle comprend, bien entendu, le procédé de dismutation utilisant ce catalyseur.

La dismutation ou métathèse d'oléfines, bien que technique relativement jeune, existant depuis seulement une vingtaine d'années, a donné lieu à un grand nombre de travaux; comme elle réside en l'échange de groupements aboutissant à la double liaison, entre deux molécules d'oléfine, on a la possibilité de produire des composés difficiles à synthétiser par des voies connues, d'où le grand intérêt industriel de ce mode de réaction. L'opération s'effectuant par catalyse hétérogène ou homogène, les systèmes catalytiques généralement employés sont à base de métaux de transition, W, Mo ou Re. La réaction peut s'écrire schématiquement comme suit:

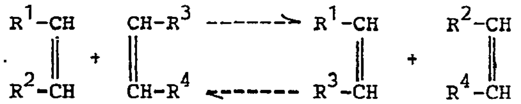 (1)

$R^1$ à $R^4$ désignant des chaînes ou groupes carbonés, dont certains peuvent être semblables, pouvant porter des fonctions telles que, par exemple, carboxyles, hydroxyles, amines, nitriles, silyles, halogènes, éthers ou autres.

Dans le cas de la catalyse homogène, on emploie le plus souvent des systèmes comprenant un composé tel que $WCl_6$, $W(CO)_5Cl$, $W(CO)_4Cl_2$, $MoCl_5$, $Mo(NO)_2Cl_2$, $ReCl_5$, etc., en combinaison avec un organométallique du type LiR, RMgX, $AlR_3$, $R_2AlCl$, $RAlCl_2$, $SnR_4$, $NaBH_4$ ou similaire. D'après certains travaux récents (H.T. Dodd et K.J. Rutt, «Journal of Molecular Catalysis», 15, 1982, pp. 103-110), des systèmes catalytiques particulièrement intéressants comprendraient des dichlorures de tétraphénoxytungstène associés à des organoaluminiques, notamment à $C_2H_5AlCl_2$ ou à $(C_2H_5)_3Al_2Cl_3$. Selon leurs auteurs, de tels systèmes conviendraient bien à la métathèse d'oléfines ne portant pas de groupes fonctionnels, par exemple à celle du pentène-2, à condition que le rapport molaire W/alkène soit supérieur à 1/100, et que W/Al soit aussi voisin que possible de 1/6. Il résulte d'autres travaux (J. Otton, Y. Colleulle et J. Varagnat, «Journal of Molecular Catalysis», 8, 1980, pp. 313-324) que les meilleurs résultats, dans la métathèse d'oléfines à groupes fonctionnels, s'obtiendraient avec un système catalytique formé de $WCl_6 + Sn(CH_3)4$, le rapport Sn/W étant de 2.

Malgré tous les travaux antérieurs, les applications industrielles de la métathèse d'oléfines souffrent encore de certaines insuffisances et demandent des perfectionnements. En particulier, la réaction est généralement trop lente; d'autre part, bien que les composants du catalyseur soient récupérables, il serait tout de même préférable de ne pas avoir à employer de fortes teneurs en W et en Al ou en Sn dans le milieu réactionnel, afin de réduire les frais de préparation et de récupération du système catalytique. Or, comme on l'a vu plus haut, selon la technique actuelle il faut des quantités substantielles de catalyseur et de cocatalyseur. En outre, les catalyseurs à base de $WCl_6$ sont sensibles aux traces d'eau et d'oxygène; il en résulte un manque de constance de l'activité catalytique, d'où mauvaise reproductibilité des réactions.

La présente invention apporte un perfectionnement marqué en ce qu'elle permet d'obtenir des vitesses de réaction fortement accrues, même avec des proportions de catalyseur tungstique moindres. Elle rend en outre la métathèse beaucoup moins dépendante du rapport W/Al ou W/Sn dans le système catalytique utilisé, et insensible aux traces d'eau ou/et d'oxygène.

Ces avantages sont obtenus par la modification du complexe de tungstène catalyseur d'une façon que rien ne pouvait suggérer dans la technique connue.

Ainsi le catalyseur perfectionné suivant l'invention est-il caractérisé en ce que son composé de tungstène comporte 4 atomes d'halogène liés à l'atome de W et 2 groupes phénoxy portés par ce même atome.

Ce composé peut être représenté d'une façon générale par la formule:

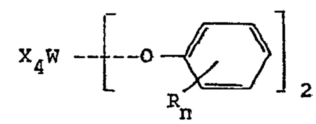 (2)

où X est un halogène, plus spécialement fluor, chlore ou brome, R étant un groupe non organique ou un atome électronégatif, notamment un halogène, $-NO_2$, $-SO_3H$, $-CN$; n peut être un nombre entier de 1 à 5, de préférence 1 à 3.

Lorsque R est un halogène, représentant d'ailleurs une forme préférée de l'invention, il peut être identique à ou différent de X. Des catalyseurs particulièrement avantageux sont de la forme:

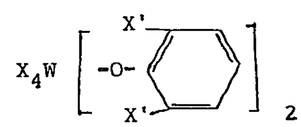 (3)

où X et X' sont des halogènes semblables ou différentss, en particulier Cl ou/et Br. La position des deux X' en ortho du groupe oxy augmente de façon inattendue l'activité du catalyseur.

Des halogènes différents, F, Cl, Br, I, peuvent constituer les R.

Il n'y a dans l'art aucun fait ou principe qui ait pu faire prévoir que les catalyseurs de formule 2 suivant l'invention seraient considérablement plus actifs que ceux de Dodd et Rutt:

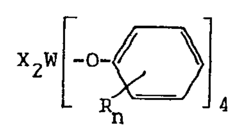

mentionnés plus haut. Il n'y avait, en effet, aucune raison de penser que le remplacement de 4 groupes phénoxy par 2 et de 2 halogènes par 4 permettrait d'accroître la vitesse de métathèse de plus de

dix fois, comme le montrent les exemples comparatifs donnés plus loin.

Le diphénoxy tétrahalogéno tungstène suivant l'invention (formule 2) est employé en association avec tout cocatalyseur connu pour la métathèse, notamment avec un de ceux qui sont mentionnés plus haut à propos de la technique connue. Lorsqu'il s'agit de la métathèse d'oléfines ne portant pas de groupes fonctionnels, le cocatalyseur préféré est un dérivé organoaluminique ou un halogénure d'un tel dérivé. Ainsi conviennent des composés $R_3Al$, $RAlX_2$, $R_2AlX$ ou/et $R_3Al_2X_3$, où R est de préférence un alkyle en $C_1$ à $C_6$ et X est chlore ou brome.

Cependant, le cocatalyseur à base d'organoaluminique a tendance à favoriser des réactions secondaires; cet inconvénient est évité, suivant une forme d'exécution de l'invention, par l'emploi comme cocatalyseur d'un composé organique de Sn ou de Pb; ces cocatalyseurs donnent en outre des résultats bien améliorés dans la métathèse d'oléfines porteuses de groupes fonctionnels.

Les composés organiques de l'étain, utilisables suivant l'invention, sont du type $X_nSnR_{(4-n)}$, où X est un halogène et R un alkyle, de préférence en $C_1$ à $C_6$, n étant 0 à 2.

Parmi les dérivés organiques du Pb conviennent à la réalisation de l'invention surtout ceux dont la composition répond aux formules:

$$PbR'_mX_{(4-m)} \text{ ou } Pb_2R'_mX_{(6-m)}$$

R' étant un groupe hydrocarboné, X un halogène et m de préférence 2 à 4 pour la première, et 2 à 6 pour la deuxième formule. Les R' sont le plus souvent des alkyles en $C_1$ à $C_{18}$ ou des aryles en $C_6$ à $C_{10}$, éventuellement substitués.

A titre d'indications non limitatives, voici quelques-uns des organoplombiques utilisables selon l'invention:

| | | |
|---|---|---|
| $Pb(CH_3)_4$ | $Pb(CH_3)_3F$ | $Pb(CH_3)_2Br_2$ |
| $Pb(C_2H_5)_4$ | $Pb(CH_3)_3Cl$ | $Pb(C_2H_5)_2Cl_2$ |
| $Pb(C_4H_9)_4$ | $Pb(CH_3)_3Br$ | $Pb(C_3H_7)_2Cl_2$ |
| $Pb(C_6H_5)_4$ | $Pb(CH_3)_3I$ | $Pb(C_5H_{11})_2Br_2$ |
| $Pb_2(CH_3)_6$ | $Pb(C_6H_5)_3Cl$ | $Pb(C_6H_5)_2Cl_2$ |
| $Pb_2(C_2H_5)_6$ | $Pb(C_6H_5)_3Br$ | $Pb(C_8H_{17})_2Br_2$ |
| $Pb_2(C_6H_5)_6$ | $Pb(C_4H_9)_3Cl$ | |
| $Pb_2(C_6H_{11})_6$ | $Pb(CH_3)_2Cl_2$ | |

Pour constituer le système catalytique suivant l'invention, on choisit ceux des composés du Pb qui présentent une certaine solubilité dans des solvants organiques utilisables dans les milieux réactionnels de métathèse.

Des plombs-tétraalkyles, notamment d'alkyles en $C_1$ à $C_6$, sont bien accessibles industriellement et donnent d'excellents résultats dans la dismutation d'oléfines, suivant l'invention; leur emploi conjoint avec les complexes (2) du tungstène constitue donc une forme d'exécution particulièrement pratique de l'invention.

Contrairement à l'art connu, l'équilibre selon la réaction 1 est atteint rapidement, même avec des rapports atomiques Al/W très différents de 6, ce qui procure de la souplesse à la marche industrielle du procédé. En effet, les rapports atomiques Sn/W ou Pb/W dans les systèmes catalytiques suivant l'invention peuvent être de l'ordre de 0,5 à 5, de préférence entre 1 et 3 et, le mieux, de 1,5 à 2,5 avec un optimum près de 2.

Lorsque la métathèse concerne les oléfines porteuses de groupes fonctionnels, le meilleur cocatalyseur semble être – comme indiqué dans la littérature technique – un tétraalkylétain, notamment un $SnR'_4$ où R' est un alkyle, de préférence en $C_1$ à $C_6$. Mais alors, suivant l'invention, le système catalytique n'est plus $WCl_6 + SnR'_4$ comme dans la technique antérieure, mais:

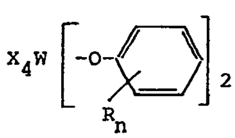 + $SnR'_4$ ou $PbR'_4$

et l'équilibre de la transformation des oléfines fonctionnelles est atteint beaucoup plus vite.

Les catalyseurs perfectionnés suivant l'invention s'appliquent avantageusement aux différents types de réaction auxquelles peut donner lieu la métathèse d'oléfines. On peut trouver la description de telles réactions par exemple dans l'article de Jean Otton, publié par «Informations Chimie», N° 201, mai 1980, pp. 161-168. Aussi les exemples d'applications donnés ci-après ne sont-ils en rien limitatifs des possibilités des nouveaux catalyseurs.

*Exemple 1:*

*Préparation d'un catalyseur perfectionné suivant l'invention (A)*

Dans un réacteur tricol, surmonté d'un réfrigérant et balayé par un courant d'argon sec, on introduit 2,5 g de $WCl_6$ ($6,3 \times 10^{-3}$ mol), 2,05 g de dichloro-2,6 phénol ($12,6 \times 10^{-3}$ mol) et 30 ml de toluène anhydre. Après chauffage à 80° C, pendant 3 h, on récupère, par évaporation du toluène, une poudre de couleur noire qui est lavée à l'éthanol. L'analyse élémentaire est conforme à la formule:

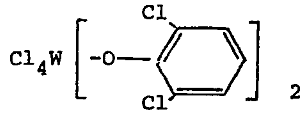

Calcul: C 22,18% H 0,93% Cl 43,66% W 28,30%
*Analyse:* C 22,3 % H 1,3 % Cl 42,7 % W 28,0 %

Ce catalyseur est désigné par A dans la suite de la présente description.

*Exemple 2:*

*Préparation d'un catalyseur de l'art antérieur (B)*

(Dodd et Rutt cités plus haut) considéré comme un des meilleurs.

Dans un réacteur tricol surmonté d'un réfrigérant et balayé par un courant d'argon sec, on introduit 2,5 g de $WCl_6$ ($6,3 \times 10^{-3}$ mol), 3,2 g de p-chlorophénol ($25,2 \times 10^{-3}$ mol) et 30 ml de toluène anhydre. Après chauffage à 80° C, pendant 3 h, on récupère, par évaporation du toluène, une poudre de couleur noire qui, après lavage à l'éthanol, est recristallisée dans le mélange toluène/pentane. L'analyse élémentaire est conforme à la formule

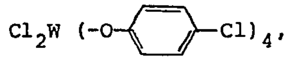

c'est-à-dire tétra(para-chlorophénoxy)dichlorotungstène.

Calcul: C 37,65% H 2,10% Cl 27,84% W 24,5%

*Analyse:* C 37,3 % H 2,2 % Cl 27,7 % W 23,4%

Le catalyseur est appelé B dans les exemples suivants.

*Exemples 3 à 6:*

Les essais consistent à effectuer la métathèse du cispentène-2 suivant la réaction

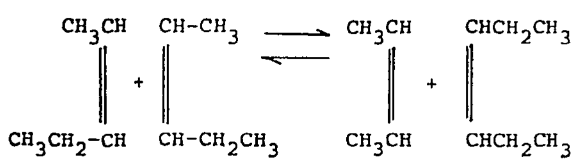

donnant du butène-2 et de l'hexène-3.

Dans un réacteur pour travail discontinu, préalablement purgé à l'argon, on introduit d'abord la quantité de composé tungsténique (A ou B) indiquée au tableau des résultats; ensuite, on y verse 60 ml de chlorobenzène servant de solvant. A la solution ainsi formée on ajoute $6 \times 10^{-4}$ mol de $C_2H_5AlCl_2$.

Après 5 min d'agitation des réactifs, le cis-pentène-2 est introduit dans le réacteur en une quantité qui est précisée sous la forme du nombre de moles de pentène par atome de W présent.

Le réacteur est laissé à la température ambiante. A des intervalles de temps déterminés, on analyse la phase gazeuse pour connaître la proportion de butène formé.

Il est avéré qu'à 20° C, lorsque l'équilibre thermodynamique est réalisé, le pentène-2 initial est transformé en 25% de butène-2, 25% d'hexène-3 et 50% de pentène-2.

Le tableau I indique les pourcentages de butène produit après des temps variables pour chacun des exemples 3 à 6.

*(Tableau en tête de la colonne suivante)*

On peut constater que le catalyseur A suivant l'invention conduit à des vitesses de réaction considérablement accrues par rapport à celle que fournit le catalyseur B de l'art antérieur (exemple 6). En effet, l'équilibre est atteint après:

*Tableau I*

| Exemple N° | 3 | 4 | 5 | 6 |
|---|---|---|---|---|
| Catalyseur | A | A | A | B |
| n-mol × $10^{-6}$ | 7 | 14 | 140 | 100 |
| Moles oléfine par atome W | 3600 | 360 | 36 | 50 |
| Atomes W pour 100 mol d'oléfine | 0,028 | 0,28 | 2,8 | 2 |
| Al/W | 86 | 42,8 | 4,3 | 6 |
| Rendement en butène (mol-%) après | | | | |
| 5 min | 15,0 | 21,7 | 25,0 | 2,2 |
| 10 min | 20,3 | 24,9 | 25,0 | 3,7 |
| 20 min | 23,4 | 25,0 | 25,0 | 6,2 |
| 45 min | 24,0 | 25,0 | 25,0 | 10,0 |
| 120 min | 25,0 | 25,0 | 25,0 | 14,7 |
| 300 min | 25,0 | 25,0 | 25,0 | 24,8 |

45 min dans l'exemple 3
10 min dans l'exemple 4
5 min dans l'exemple 5

alors qu'il faut 5 h (300 min) avec le catalyseur B. L'exemple 5 est particulièrement intéressant par l'extrême vitesse de la métathèse, mais même l'exemple 3 est encore industriellement tout à fait viable, surtout en comparaison de l'exemple 6. Il est donc possible d'employer le catalyseur perfectionné A avec des rapports oléfine/W et Al/W très variables, tout en restant dans une marge de vitesses de réaction parfaitement acceptables industriellement.

*Exemple 7:*

*Changement de cocatalyseur*

Les opérations de l'exemple 5 sont répétées avec les mêmes proportions de matières, mais le cocatalyseur est constitué par du sesquichlorure de méthylaluminium, soit $3 \times 10^{-4}$ mol de $(CH_3)_3Al_2Cl_3$.

L'équilibre est pratiquement atteint après 10 min, avec 24,6% de butène-2 formé; en 5 min, il y a déjà 24% de butène; on retrouve donc à peu de choses près les résultats de l'exemple 5 où le cocatalyseur était $C_2H_5AlCl_2$.

*Exemple 8:*

*Essai d'un catalyseur tungstique à ligande de phénoxy bromé (C)*

Par un procédé analogue à celui de l'exemple 1, on prépare le composé complexe de W de formule:

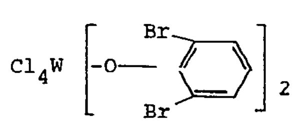

(appelé C dans l'exemple 10)

La métathèse de cis-pentène-2 est effectuée de la même manière qu'à l'exemple 4, avec $2,8 \times 10^{-5}$ mol (28 mg) de composé C pour $6 \times 10^{-4}$ mol

de $C_2H_5AlCl_2$ dans 60 ml de chlorobenzène. Rapport molaire oléfine/W = 360, Al/W = 21,4.

Après 5 min à la température ambiante, on trouve un rendement en butène de 24% et, après 30 min, 24,3%, donc résultats équivalents à ceux de l'exemple 4 où le groupe phénoxy du catalyseur A est dichloré.

*Exemples 9 à 11:*

*Métathèse d'une oléfine porteuse de groupes fonctionnels (oléate d'éthyle)*

Les essais consistent à maintenir à 90° C, en présence d'un système catalytique, constitué par un composé du W et de l'étain tétraméthyle, de l'oléate d'éthyle, et à déterminer le pourcentage d'octadécène-9 formé suivant la réaction:

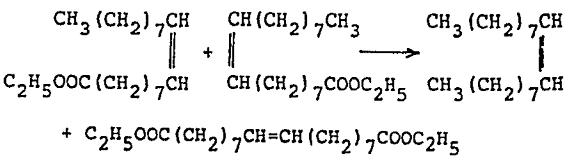

qui fournit, en même temps, de l'hexadécène-8 di-1,16-carboxylate d'éthyle.

La réaction est réalisée dans un appareil pour travail discontinu, purgé préalablement à l'argon, dans lequel on introduit $10^{-4}$ mol de composé tungstique, ensuite 5 ml de chlorobenzène en tant que solvant, et enfin $2 \times 10^{-4}$ mol de $Sn(CH_3)_4$.

Après 15 min d'agitation de ces réactifs, à 90° C, $0,5 \times 10^{-2}$ mol d'oléate d'éthyle est introduit dans le réacteur.

Le rapport molaire oléfine/W est ainsi de 50, et Sn/W=2. A certains intervalles de temps, on analyse la phase liquide du réacteur pour déterminer le rendement en octadécène-9, c'est-à-dire le rapport en pourcentage du nombre de moles de ce composé formé au nombre de moles d'oléate mis en œuvre. Le tableau II donne les résultats obtenus avec trois catalyseurs différents: A et C suivant l'invention (formules données dans les exemples 1 et 8), ainsi que $WCl_6$, le plus employé dans la technique antérieure pour ce genre de métathèse.

*Tableau II*

| Exemple N° | 9 | 10 | 11 |
|---|---|---|---|
| Catalyseur | A | C | $WCl_6$ |
| Rendement en octadécène (mol-%), après: | | | |
| ½ h | 6 | 10 | 4,2 |
| 1 h | 9 | 11,5 | — |
| 2 h | 12 | — | 5 |
| 4 h | 13,3 | 14,5 | — |
| 20 h | 14,5 | — | 6 |

La remarquable activité des catalyseurs suivant l'invention se confirme ici, puisque le catalyseur A fournit, déjà après 2 h, le double du rendement (12%) que l'on obtient avec le classique $WCl_6$ (6%) après 20 h seulement. Particulièrement intéressant apparaît le catalyseur C (Br dans le phénoxy), puisqu'il conduit à 14,5% après à peine 4 h.

*Exemple 12:*

Application du catalyseur de l'invention:

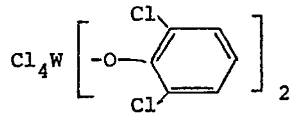

associé à $SnMe_4$, à la réaction de métathèse croisée entre des acétates d'alkényles-ω et des oléfines. C'est une voie de synthèse de phéromones d'insectes. La réaction étudiée est:

$CH_2=CH(CH_2)_3OAc + CH_3CH_2CH=CHCH_2CH_3$

I ⇅ II

$AcO(CH_2)_3CH=CH(CH_2)_3OAc + CH_3CH_2CH=CH(CH_2)_3OAc + CH_2=CH_2$

III IV V

$+ CH_3CH_2CH=CH_2$

VI

Dans une expérience typique, la réaction de métathèse croisée entre l'acétate de pentène-4 yl-1 et l'hexène-3 a été réalisée dans un appareil de type discontinu, convenablement purgé à l'argon. Les réactifs ont été introduits dans l'ordre suivant:

- 65 mg ($1,10^{-4}$ mol) de 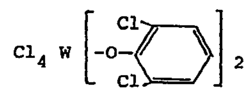
- 5 ml de chlorobenzène (solvant)
- $2,10^{-4}$ mol de $SnMe_4$

Après 20 min d'agitation des réactifs à 65° C, l'acétate de pentène-4 yl-1 ($0,25 \times 10^{-2}$ mol) et l'hexène-3 ($0,25 \times 10^{-2}$ mol) sont introduits dans le réacteur; rapport (oléfine + acétate)/W = 50 mol/mol. L'analyse de la phase liquide, effectuée au cours du temps, montre qu'après 1 h de réacteur à 65° C, on a converti 32% de l'acétate de départ et 80% après 20 h.

Le tableau III rapporte ces résultats à côté de ceux d'une préparation du même produit par le procédé de l'art antérieur utilisant $WCl_6$+$SnMe_4$ comme catalyseur [J. Levisalles et D. Villemin, «Tetrahedron», *36* (1980) 3181].

*(Tableau en tête de la page suivante)*

La sélectivité en produit IV s'entend par rapport à l'acétate de départ I.

Les résultats obtenus montrent que, pour cette réaction, le catalyseur relevant de la présente invention se montre plus actif que le catalyseur classique $WCl_6$ + $SnMe_4$ dont l'utilisation dans la même réaction a été décrite dans la littérature citée ci-dessus.

Ainsi le procédé de l'invention présente-t-il en particulier une utilité pour la fabrication de phéromones d'insectes, dont l'intérêt industriel va croissant à l'heure actuelle.

*Exemple 13:*

Emploi du même système catalytique que dans l'exemple 12 pour la réaction de métathèse croisée entre deux oléfines terminales: le décène-1 et le pentadécène-1. Cette réaction conduit à la formation du tricosène-9 dont l'isomère cis est la phéromone de la Musca domestica:

*Tableau III*

| Catalyseur | Température (°C) | Rapport molaire (I+II) : W | Temps de réaction (h) | Conversion de I (%) | Sélectivité en composé IV (%) |
|---|---|---|---|---|---|
| $WCl_6$+$SnMe_4$ Sn/W = 2 | 70 | 10 | 16 | 57,5 | 37 |
| 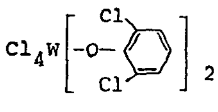 + $SnMe_4$ Sn/W = 2 | 65 | 50 | 1<br>4<br>20 | 32<br>52<br>80 | 62<br>75<br>75 |

$$CH_2{=}CH(CH_2)_7CH_3 + CH_2{=}CH(CH_2)_{12}CH_3$$
$$\rightleftharpoons$$
$$CH_3(CH_2)_7CH{=}CH(CH_2)_{12}CH_3 + CH_2{=}CH_2$$

tricosène

$$+CH_3(CH_2)_7CH{=}CH(CH_2)_7CH_3 \quad + CH_3(CH_2)_{12}CH{=}CH(CH_2)_{12}CH_3$$

Une réaction de métathèse croisée entre le décène-1 et le pentadécène-1 a été réalisée dans un appareil de type discontinu convenablement purgé à l'argon. Les réactifs ont été introduits dans l'ordre suivant:

– 65 mg ($1{,}10^{-4}$ mol) de 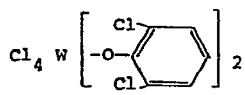
– 5 ml de chlorobenzène (solvant)
– $2{,}10^{-4}$ de $SnMe_4$

Après 20 min d'agitation des réactifs à 65° C, le décène-1 ($0{,}5 \times 10^{-2}$ mol) et le pentadécène-1 ($0{,}5 \times 10^{-2}$ mol) (oléfines/W = 100 mol/mol) sont introduits dans le réacteur. L'analyse de la phase liquide, effectuée au cours du temps, montre qu'après 90 min de réaction à 65° C, on obtient un rendement de 23% en poids de tricosène-9. Les résultats sont réunis au tableau IV comparativement à ceux que donnent les catalyseurs de la technique les plus efficaces [F.W. Kupper et R. Streck, «Z. Naturforsch.», *31* (b) (1976) 1256].

Ces résultats montrent que le catalyseur de la présente invention permet d'atteindre des rendements en tricosène-9 plus élevés que ceux qui sont obtenus avec des catalyseurs tels que $WCl_6/EtOH/EtAlCl_2$ ou $Mo(NO)_2Cl_2(PPh_3)_2/Me_3Al_2Cl_3$; ce dernier système est pourtant décrit dans la littérature comme particulièrement adapté à la métathèse des oléfines terminales [E.A. Zuech, W.B. Hugues, D.H. Kubicek et E.T. Kittleman, «J. Am. Chem. Soc.», *92* (1970) 528].

*Exemples 14 et 15:*

*Métathèse d'une oléfine porteuse de groupes fonctionnels (oléate d'éthyle) avec le catalyseur A associé à $SnBu_4$ ou $PbBu_4$.*

Les essais consistent à maintenir à 85° C, en présence d'un système catalytique constitué par du catalyseur A et de l'étain tétrabutyle ou du plomb tétrabutyle, de l'oléate d'éthyle, et à déterminer le pourcentage d'octadécène-9 formé suivant la réaction:

*Tableau IV*

| Catalyseur | Oléfines de départ (ml) | Température (°C) | Temps de réaction (min) | Rdt (% poids) en tricosène-9 |
|---|---|---|---|---|
| $WCl_6/EtOH/EtAlCl_2$<br>0,1 0,3 0,5 (mmol) | octadécène-9/hexadécène-2<br>20 4,4 | 50 | 5<br>15<br>30 | 8,6<br>11,8<br>11,8 |
| $Mo(NO)_2Cl_2(PPh_3)_2$/<br>0,05<br>$Me_3Al_2Cl_3$ [a]<br>0,6 (mmol) | décène-1/pentadécène-1<br>1 1,35 | 20 | 15<br>135<br>1230 | 6,9<br>8,7<br>12,0 |
| 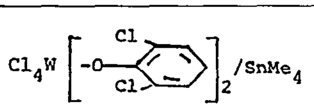<br>0,1 0,2 (mmol) | décène-1/pentadécène-1<br>1 1,35 | 65 | 15<br>45<br>90<br>180 | 10,1<br>16,6<br>23,0<br>23,0 |

[a] Catalyseur et cocatalyseur mis en solution dans 15 ml de chlorobenzène sous argon; interaction pendant 15 min, puis introduction des oléfines.

$2\ CH_3(CH_2)_7CH{=}CH(CH_2)_7COOEt \rightleftarrows CH_3(CH_2)_7CH{=}CH(CH_2)_7CH_3 + EtOOC(CH_2)_7CH{=}CH(CH_2)_7COOEt$

qui fournit, en même temps, de l'hexadécène-8-di-1, 16-carboxylate d'éthyle.

La réaction est réalisée dans un appareil pour travail discontinu, purgé préalablement à l'argon, dans lequel on introduit $2\times10^{-4}$ mol de catalyseur A, ensuite 5 ml de chlorobenzène en tant que solvant, $2\times10^{-4}$ mol de $SnBu_4$ ou $PbBu_4$ et enfin $0,5\times10^{-2}$ mol d'oléate d'éthyle. Le rapport molaire oléfine/W est ainsi de 50, et Sn/W ou Pb/W de 2. La réaction est conduite à 85° C et, à certains intervalles de temps, on analyse la phase liquide du réacteur pour déterminer le rendement en octadécène-9, c'est-à-dire le rapport en pourcentage du nombre de moles de ce composé formé au nombre de moles d'oléate mis en œuvre.

Le tableau V donne les résultats obtenus avec deux systèmes catalytiques différents: A suivant l'invention (formule donnée dans les exemples 1 à 8) associé à $SnBu_4$, d'une part, ou à $PbBu_4$, d'autre part.

*Tableau V*

| Exemple | 14 | 15 |
|---|---|---|
| Catalyseur | A + $SnBu_4$ | A + $PbBu_4$ |
| Rendement en octadécène (mol-%) après: | (%) | (%) |
| ½ h | 0 | 14 |
| 1 h | 0 | 14 |
| 4 h | 5 | — |
| 18 h | 14 | — |

La comparaison de ces exemples 14 et 15 avec les exemples 9, 10 et 11 du texte précédent montre que, dans cette réaction, les catalyseurs suivant l'invention sont plus actifs que le catalyseur classique $WCl_6$, que l'on utilise comme cocatalyseur $SnMe_4$, $SnBu_4$ ou $PbBu_4$. Un résultat remarquable est la très grande activité du catalyseur A associé à $PbBu_4$ puisqu'en 30 min on obtient déjà le maximum de rendement, alors qu'il faut 18 h pour l'atteindre avec le système A + $SnBu_4$.

*Exemple 16:*

Application du catalyseur de l'invention A associé à du $PbBu_4$ à la métathèse croisée entre des esters acides oléfiniques et des oléfines. C'est une voie de synthèse de phéromones d'insectes.

La réaction étudiée est:

$CH_3(CH_2)_7CH{=}CH(CH_2)_7COOEt + CH_3(CH_2)_3CH{=}CH(CH_2)_3CH_3$

I ⇅ II

$EtOOC(CH_2)_7CH{=}CH(CH_2)_7COOEt + CH_3(CH_2)_3CH{=}CH(CH_2)_7COOEt$

III IV

$+ CH_3(CH_2)_7CH{=}CH(CH_2)_7CH_3 + CH_3(CH_2)_3CH{=}CH(CH_2)_7CH_3$

V VI

Cette réaction, entre l'oléate d'éthyle et le décène-5, a été réalisée dans un appareil de type discontinu, convenablement purgé à l'argon. Les réactifs ont été introduits dans l'ordre suivant:

– 65 mg ($1\times10^{-4}$ mol)

$$\text{de } Cl_4W{-}\left[{-}O{-}C_6H_3Cl_2\right]_2$$

– 5 ml de chlorobenzène (solvant)

– $2\times10^{-4}$ mol de $PbBu_4$

Après 20 min d'agitation des réactifs à 85° C, l'oléate d'éthyle ($0,375\times10^{-2}$ mol) et le décène-5 ($0,75\times10^{-2}$ mol) sont introduits dans le réacteur; rapport (oléfine + oléate)/W = 112,5 mol/mol. L'analyse de la phase liquide effectuée au cours du temps montre qu'après 1 h de réaction à 85° C, on a converti 72% de l'oléate de départ. Le tableau VI rapporte ce résultat à côté d'un résultat d'une préparation du même produit par le procédé de l'art antérieur utilisant $WCl_6$ + $SnMe_4$ comme catalyseur.

*Tableau VI*

| Catalyseur | Température (° C) | Rapport molaire (I+II) : W | Temps de réaction (h) | Conversion de I (%) | Sélectivité en composé IV (%) |
|---|---|---|---|---|---|
| $WCl_6+SnMe_4$ Sn/W = 2 | 85 | 50 | 1<br>5 | 50<br>60 | 35<br>40 |
| $Cl_4W[-O-C_6H_3Cl_2]_2$ + $PbBu_4$ Pb/W = 2 | 85 | 112,5 | 1<br>2 | 72<br>72 | 66<br>66 |

La sélectivité en produit IV s'entend par rapport à l'oléate de départ I.

Les résultats obtenus montrent que, pour cette réaction, le catalyseur relevant de la présente invention se révèle plus actif que le catalyseur classique $WCl_6$ + $SnMe_4$.

*Exemples 17 et 18:*

Polymérisation par métathèse du dicyclopentadiène. Dans un appareil purgé à l'argon on procède à des opérations discontinues sur les quantités suivantes de réactifs introduits dans l'ordre:

32 mg ($0,5\times10^{-4}$ mol) du même catalyseur que dans l'exemple 14;

20 ml de chlorobenzène comme solvant
$2 \times 10^{-4}$ mol de $SnBu_4$ ou $2 \times 10^{-4}$ mol de $PbBu_4$

Après environ 5 min à 70° C, on introduit 3,4 ml, soit $2,5 \times 10^{-2}$ mol, de dicyclopentadiène. La réaction est poursuivie à 70° C durant 2 h.

On trouve alors les rendements en polymère suivants:

Exemple 17 ... cocatalyseur $SnBu_4$ 15%
Exemple 18 ... cocatalyseur $PbBu_4$ 45%

*Exemple 19:*

Application du catalyseur de l'invention:

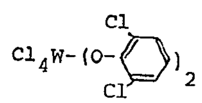

(désigné plus haut par A), associé à $EtAlCl_2$ ou à $Et_2AlCl$, à la polymérisation par métathèse d'une oléfine cyclique, le dicyclopentadiène.

Dans deux expériences typiques, la réaction de polymérisation du dicyclopentadiène a été réalisée dans un appareil de type discontinu convenablement purgé à l'argon. Les réactifs ont été introduits dans l'ordre suivant:

32 mg ($0,5 \times 10^{-4}$ mol) de catalyseur au W susindiqué
13,5 ml de dicyclopentadiène ($10^{-1}$ mol)
$6 \times 10^{-4}$ mol d'$EtAlCl_2$ ou d'$Et_2AlCl$ dans 5 ml de chlorobenzène

Le rapport molaire oléfine/W est ainsi de 2000.
Rapport cocatalyseur/catalyseur = 12; température: 25° C.

Des essais parallèles, dans des conditions identiques, sont effectués avec un catalyseur de l'art antérieur (FR-A N° 2180038) constitué par du dichlorure tétrakis-(di-isopropyl 2,6-phénate) de tungstène, désigné par D dans le tableau VII, au lieu du catalyseur suivant l'invention A.

*Résultats*

*Tableau VII*

| Cocatalyseur | Cocatalyseur / catalyseur | Temps de réaction (s) | Rendement en % polymère | |
|---|---|---|---|---|
| | | | A | D |
| $EtAlCl_2$ | 12 | 5 | 100 | 12 |
| $EtAlCl_2$ | 12 | 300 | 100 | 100 |
| $Et_2AlCl$ | 12 | 30 | 75 | — |

On voit qu'avec le catalyseur de l'art antérieur (D), la polymérisation est beaucoup plus lente: il faut 300 s là où, avec le catalyseur A, on obtient une polymérisation totale en 5 s.

*Exemple 20:*

Comparaison du catalyseur de l'invention avec un catalyseur de l'art antérieur, le dichlorure-tétrakis-(2,6-di-isopropyl phénate) de tungstène (FR N° 2180038) dans la réaction de métathèse du pentène-2-cis.

Dans un réacteur pour travail discontinu, préalablement purgé à l'argon, on introduit d'abord la quantité de composé tungsténique (catalyseur de l'invention ou catalyseur de l'art antérieur préparé de façon rigoureusement identique à la méthode décrite p. 10 du brevet FR-A N° 2180038) indiquée au tableau des résultats; ensuite, on y verse 60 ml de chlorobenzène servant de solvant. A la solution ainsi formée on ajoute $6 \times 10^{-4}$ mol de $C_2H_5AlCl_2$.

Après 5 min d'agitation des réactifs, le cis-pentène-2 est introduit dans le réacteur en une quantité qui est précisée sous la forme du nombre de moles de pentène par atome de W présent. Le réacteur est laissé à la température ambiante. A des intervalles de temps déterminés, on analyse la phase gazeuse pour connaître la proportion du butène formé. Il est avéré qu'à 20° C, lorsque l'équilibre thermodynamique est réalisé, le pentène-2 initial est transformé en 25% de butène-2, 25% d'hexène-3 et 50% de pentène-2.

Le tableau VIII indique les pourcentages de butène produit après des temps variables.

*Tableau VIII*

| Catalyseur | $Cl_4W-[O-C_6H_3Cl_2]_2$ (même qu'à l'exemple 14) | Dichlorure-tétrakis-(2,6-di-isopropyl phénate) de tungstène (art antérieur) |
|---|---|---|
| n-mol $\times 10^{-6}$ | 140 | 100 |
| Moles d'oléfine par atome W | 36 | 50 |
| Atomes de W pour 100 mol d'oléfine | 2,8 | 2 |
| Al/W | 4,3 | 6 |
| Rendement en butène (mol-%) après: | | |
| 5 min | 25,0 | 0,7 |
| 20 min | 25,0 | 1,0 |
| 120 min | 25,0 | 1,5 |

On peut constater que le catalyseur de l'invention conduit, dans la métathèse du pentène-2-cis, à une vitesse de réaction considérablement accrue par rapport à celle que fournit le catalyseur de la technique connue.

Il est à noter que le composé A décrit dans l'exemple 1 est un produit chimique nouveau.

## Revendications

1. Catalyseur pour la métathèse d'oléfines, qui comprend une combinaison du tungstène avec des atomes d'halogène et des groupes phénoxy pouvant porter des substituants, caractérisé en ce que la combinaison du tungstène répond à la formule:

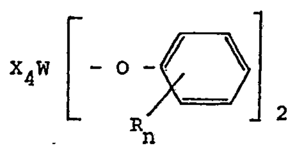

où X est un halogène, R un groupe ou atome électronégatif et n un nombre entier de 1 à 5.

2. Catalyseur suivant la revendication 1, dans lequel X est Cl ou Br, R étant un halogène, en particulier Cl ou Br, caractérisé en ce que n est au moins 2.

3. Catalyseur suivant l'une des revendications 1 ou 2, caractérisé en ce qu'il y a plusieurs halogènes R différents.

4. Catalyseur suivant l'une des revendications 1 à 3, caractérisé en ce que le groupe phénoxy porte un atome d'halogène en position 2 et un en position 6 par rapport au carbone du groupe oxy.

5. Catalyseur suivant la revendication 1, caractérisé en ce que le groupe électronégatif est $-NO_2$, $-SO_3H$ ou $-CN$.

6. Procédé de métathèse d'oléfines, dans lequel la ou les oléfines sont maintenues au contact d'un système catalytique comprenant une combinaison du tungstène avec des atomes d'halogène et des groupes phénoxy portant des substituants, et un dérivé organique de l'aluminium, de l'étain ou du plomb, caractérisé en ce que cette combinaison comporte 4 atomes d'halogène et 2 groupes phénoxy, substitués avec un ou plusieurs groupes ou atomes électronégatifs, liés à un atome de tungstène.

7. Procédé suivant la revendication 6, dans lequel le composé de W est associé à un organoaluminique $R_3Al$, $RAlX_2$, $R_2Al_X$ ou/et $R_3Al_2X_3$, où R est un alkyle en $C_1$ à $C_6$ et X chlore ou brome, caractérisé en ce que le rapport atomique Al/W est de préférence de 4 à 86, pour la métathèse d'oléfines sans groupes fonctionnels.

8. Procédé suivant la revendication 6, caractérisé en ce que le composé de W est associé à un organostannique $SnR'_4$, R' étant un alkyle en $C_1$ à $C_6$, pour la métathèse d'oléfines porteuses de groupes fonctionnels.

9. Procédé suivant la revendication 6, caractérisé en ce que le dérivé organique du plomb répond à la formule $PbR'_mX_{(4-m)}$ ou $Pb_2R'_mX_{(6-m)}$ où R' est un groupe hydrocarboné, X un halogène et m un nombre 2 à 4 pour la première et 2 à 6 pour la deuxième de ces formules.

10. Procédé suivant la revendication 9, caractérisé en ce que R' est un alkyle en $C_1$ à $C_{18}$.

11. Procédé suivant la revendication 9, caractérisé en ce que R' est un alkyle en $C_1$ à $C_6$ et $(4-m)$ ou $(6-m) = 0$.

12. Nouveau produit chimique constitué par une combinaison du tungstène avec le chlore et un groupe phénoxy substitué, caractérisé par la formule:

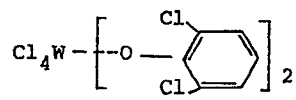

## Claims

1. Catalyst for the metathesis of olefins which comprises a combination of tungsten with halogen atoms and phenoxy groups which can carry substituents, characterized in that the tungsten combination corresponds to the formula:

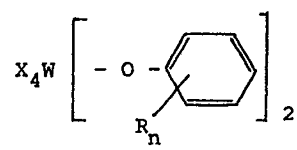

where X is a halogen, R an electronegative groupe or atom and n is an integral number from 1 to 5.

2. Catalyst according to Claim 1, in which X is Cl or Br, R being a halogen, in particular Cl or Br, characterized in that n is at least 2.

3. Catalyst according to any of Claims 1 or 2, characterized in that there are several different halogens R.

4. Catalyst according to any of Claims 1 to 3, characterized in that the phenoxy group carries a halogen atom in the 2 position and one in the 6 position with respect to the carbon of the oxy group.

5. Catalyst according to Claim 1, characterized in that the electronegative groupe is $-NO_2$, $-SO_3H$ or $-CN$.

6. Process of metathesis of olefins in which the olefin or olefins is/are maintained in contact with a catalytic system comprising a combination of tungsten with halogen atoms and phenoxy groups carrying substituents and an organic derivative of aluminium, tin or lead, characterized in that this combination comprises 4 halogen atoms and 2 phenoxy groups substituted with one or more electronegative groups or atoms connected to a tungsten atom.

7. Process according to Claim 6, in which the W compound is associated with an organo-aluminium compound $R_3Al$, $RAlX_2$, $R_2AlX$ and/or $R_3Al_2X_3$, where R is a $C_1$ to $C_6$ alkyl and X is chlorine or bromine, characterized in that the atomic ratio Al/W is preferably from 4 to 86, for the metathesis of olefins without functional groups.

8. Process according to Claim 6, characterized in that the W compound is associated with an organo-t in compound $SnR'_4$, R' being a $C_1$ to $C_6$

alkyl, for the metathesis of olefins carrying functional groups.

9. Process according to Claim 6, characterized in that the organic lead derivative corresponds to the formula $PbR_mX_{(4-m)}$ or $Pb_2R_mX_{(6-m)}$, where R' is a hydrocarbon group, X is a halogen and m is a number from 2 to 4 for the first and 2 to 6 for the second of these formulae.

10. Process according to Claim 9, characterized in that R' is a $C_1$ to $C_{18}$ alkyl.

11. Process according to Claim 9, characterized in that R' is a $C_1$ to $C_6$ alkyl and (4−m) or (6−m) = 0.

12. New chemical product constituted by a combination of tungsten with chlorine and a substituted phenoxy group, characterized by the formula:

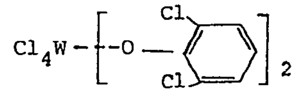

## Patentansprüche

1. Katalysator zur Umlagerung von Olefinen, der eine Kombination von Wolfram mit Halogenatomen und Phenoxygruppen, die Substituenten tragen können, umfasst, dadurch gekennzeichnet, dass die Kombination von Wolfram der Formel

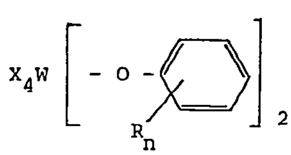

entspricht, worin X ein Halogen ist, R eine elektronegative Gruppe oder Atom ist und n eine ganze Zahl von 1 bis 5 ist.

2. Katalysator nach Anspruch 1, in dem X die Bedeutung von Cl oder Br hat, R ein Halogen ist, insbesondere Cl oder Br, dadurch gekennzeichnet, dass n mindestens 2 ist.

3. Katalysator nach einem der Ansprüche 1 oder 2, dadurch gekennzeichnet, dass er mehrere verschiedene Halogene R aufweist.

4. Katalysator nach einem der Ansprüche 1 bis 3, dadurch gekennzeichnet, dass die Phenoxygruppe ein Halogenatom in der 2-Stellung und eines in der 6-Stellung, bezogen auf den Kohlenstoff der Oxygruppe, trägt.

5. Katalysator nach Anspruch 1, dadurch gekennzeichnet, dass die elektronegative Gruppe $-NO_2$, $-SO_3H$ oder −CN ist.

6. Verfahren zur Umlagerung von Olefinen, bei dem das oder die Olefine im Kontakt mit einem Katalysatorsystem gehalten werden, das eine Kombination von Wolfram mit Halogenatomen und Phenoxygruppen, die Substituenten tragen, und ein organisches Aluminium-, Zinn- oder Bleiderivat umfasst, dadurch gekennzeichnet, dass diese Kombination 4 Halogenatome und 2 Phenoxygruppen, substituiert mit einer oder mehreren elektronegtiven Gruppen oder Atomen, gebunden an ein Wolframatom, umfasst.

7. Verfahren nach Anspruch 6, bei dem die Verbindung on W assoziiert ist mit einer Organoaluminiumverbindung $R_3Al$, $RAlX_2$, $R_2AlX$ oder/und $R_3Al_2X_3$, worin R ein Alkyl mit $C_1$ bis $C_6$ ist und X Chlor oder Brom ist, dadurch gekennzeichnet, dass das Atomverhältnis Al/W vorzugsweise 4 bis 86 beträgt, zur Umlagerung von Olefinen ohne funktionelle Gruppen.

8. Verfahren nach Anspruch 6, dadurch gekennzeichnet, dass die Verbindung von W assoziiert ist mit einer Organozinn-IV-Verbindung $SnR'_4$, worin R' ein Alkyl mit $C_1$ bis $C_6$ ist, zur Umlagerung von Olefinen, die funktionelle Gruppen tragen.

9. Verfahren nach Anspruch 6, dadurch gekennzeichnet, dass das organische Bleiderivat der Formel $PbR'_mX_{(4-m)}$ oder $Pb_2R'_mX_{(6-m)}$ entspricht, worin R' eine Kohlenwasserstoffgruppe ist, X ein Halogen ist und m eine Zahl von 2 bis 4 für die erste und von 2 bis 6 für die zweite dieser Formeln ist.

10. Verfahren nach Anspruch 9, dadurch gekennzeichnet, dass R' ein Alkyl mit $C_1$ bis $C_{18}$ ist.

11. Verfahren nach Anspruch 9, dadurch gekennzeichnet, dass R' ein Alkyl mit $C_1$ bis $C_6$ und (4−m) oder (6−m) = 0 ist.

12. Neues chemisches Produkt, gebildet aus einer Kombination von Wolfram mit Chlor und einer substituierten Phenoxygruppe, gekennzeichnet durch die Formel